# EUROPÄISCHE PATENTSCHRIFT

(11) **EP 1 767 139 B1**
(45) Veröffentlichungstag und Bekanntmachung des Hinweises auf die Patenterteilung: **30.07.2008**
(21) Anmeldenummer: 06018615.2
(22) Anmeldetag: 06.09.2006
(51) Int. Cl.: A61B 1/06, A61B 1/005

(54) **Beleuchtungssystem für endoskopische Untersuchungen**
Illumination system for endoscopy
Systeme d'eclairage pour endoscopie

(30) Priorität: 23.09.2005 DE 102005045729
(43) Veröffentlichungstag der Anmeldung: 28.03.2007
(73) Patentinhaber: Karl Storz GmbH & Co. KG, 78532 Tuttlingen (DE)
(72) Erfinder: Irion, Klaus M., Dr., 78576 Liptingen (DE)
(74) Vertreter: Hofmeister, Frank

(56) Entgegenhaltungen:
- EP-A- 1 685 791
- WO-A2-20/06037034
- US-A1- 2005 049 462
- US-A1- 2005 234 296

## Beschreibung

Die Erfindung betrifft einen Endoskopschaft mit einem Beleuchtungssystem bestehend aus einer mindestens zwei LED-Elemente aufweisenden Beleuchtungseinheit zur Ausleuchtung eines mittels einer Endoskopoptik zu betrachtenden Untersuchungsgebiets.

Der Einsatz von Endoskopen hat sich in der Chirurgie seit vielen Jahren erfolgreich bewährt und dient in vielen Fällen als ein minimal invasives Alternativverfahren zur herkömmlichen Offenen Chirurgie. Um mittels der Endoskopoptik, die beispielsweise klassisch als Relaislinsensystem, als Faserbildleiter oder auch als elektronischer Bildsensor-Chip (CMOS, CCD) zur Video-Bildaufnahme ausgelegt sein kann das Einsatzgebiet untersuchen zu können und dort operativ tätig zu werden, ist es zwingend notwendig das Untersuchungsgebiet so gut wie möglich auszuleuchten. Neben der Verwendung in der Human- und Veterinärmedizin haben sich endoskopische Untersuchungen auch in technischen Bereichen, beispielsweise zur Untersuchung von Hohlräumen, bewährt. Auch bei diesen Anwendungsfällen ist eine gute Ausleuchtung des Untersuchungsgebietes unentbehrlich für eine gute Darstellung über die Endoskopoptik.

Üblicherweise erfolgt die Beleuchtung des Untersuchungsgebiets über einen Lichtleiter, bestehend aus Glasfaserbündeln, über die Licht von einer externen Lichtquelle in das Untersuchungsgebiet geleitet wird. Da diese Beleuchtungssysteme, beispielsweise hinsichtlich der Kühlung und Beschaltung, technisch sehr aufwendig sind, ist es seit einiger Zeit bekannt, mit LED-Elementen versehene Beleuchtungssysteme zu verwenden, die am distalen Ende des Endoskopschaftes angebracht sind. Vorteil dieser am distalen Ende angeordneten LED-Beleuchtungssysteme ist das Vermeiden von Einkopplungsverlusten in den Lichtleiter sowie die lange Lebensdauer der LED-Elemente.

Die Verwendung eines Beleuchtungssystem für endoskopische Untersuchungen mit einer LED-Elemente aufweisenden Beleuchtungseinheit ist beispielsweise aus der DE 100 61 107 A1 bekannt. Nachteilig bei den bekannten Beleuchtungssystemen ist jedoch die den LED-Elementen eigene geringe zu erzielende Leistungsdichte, so dass auch bei spezieller Anordnung der LED-Elemente an der distalen Stirnseite des Endoskopschaftes nur eine nicht für alle Anwendungsfälle ausreichende Ausleuchtung des Untersuchungsgebiets zu erzielen ist.

Davon ausgehend liegt der Erfindung die **Aufgabe** zugrunde, ein Beleuchtungssystem für endoskopische Untersuchungen der eingangs genannten Art zu schaffen, das eine stets ausreichende Ausleuchtung des Untersuchungsgebietes gewährleistet.

Die **Lösung** dieser Aufgabenstellung ist erfindungsgemäß dadurch gekennzeichnet, dass die LED-Elemente der Beleuchtungseinheit so angeordnet sind, dass die Abstrahlrichtung der Beleuchtungseinheit zwischen einer Richtung im Wesentlichen senkrecht zur Betrachtungsrichtung der Endoskopoptik beim Einführen in das Untersuchungsgebiet und einer Richtung im Wesentlichen in Betrachtungsrichtung der Endoskopoptik nach dem Einführen in das Untersuchungsgebiet verschwenkbar ist.

Durch die erfindungsgemäße Verlagerung der LED-Elemente in eine Richtung im Wesentlichen senkrecht zur Betrachtungsrichtung der Endoskopoptik beim Einführen in das Untersuchungsgebiet ist erstmalig möglich bei der Verwendung von LED-Elementen zur Ausleuchtung eines endoskopischen Untersuchungsgebiets, die LED-Elemente nicht an der distalen Stirnfläche des Endoskops und somit auf einer äußerst begrenzten Fläche anordnen zu müssen. Das Verstellen der Beleuchtungseinheit nach dem Einführen in das Untersuchungsgebiet ermöglicht nachfolgend die ausreichende Ausleuchtung mittels der nunmehr in Betrachtungsrichtung der Endoskopoptik abstrahlenden LED-Elemente.

Gemäß einer bevorzugten Ausführungsform der Erfindung wird vorgeschlagen, dass die LED-Elemente der Beleuchtungseinheit in Reihe angeordnet sind. Diese Reihenanordnung der LED-Elemente ermöglicht eine flächige und überlagernde Ausleuchtung des Untersuchungsgebietes aus verschiedenen Einfallswinkeln, was zu einer besseren Tiefenwahrnehmung führt.

Gemäß einer praktischen Ausführungsform der Erfindung ist die Beleuchtungseinheit abseits der distalen Stirnfläche am distalen Ende eines Endoskopschaftes und somit in unmittelbarer Nähe der Endoskopoptik angeordnet. Alternativ ist ein erfindungsgemäßes Beleuchtungssystem jedoch auch als eigenständiges Instrument ausbildbar, das zusätzlich zu einer Endoskopoptik in das Untersuchungsgebiet eingeführt wird.

Weiterhin wird mit der Erfindung vorgeschlagen, dass zur Verbesserung der Ausleuchtung des Untersuchungsgebiets die Beleuchtungseinheit aus mehreren mit LED-Elementen ausgestatteten Beleuchtungs-Teileinheiten besteht, die vorzugsweise gegeneinander verschwenkbar am Endoskopschaft gelagert sind. Durch die Verwendung mehrerer Beleuchtungs-Teileinheiten, die jeweils mit LED-Elementen bestückt sind, kann die Gesamtzahl der zur Ausleuchtung zur Verfügung stehenden LED-Elemente deutlich erhöht werden. Darüber hinaus lassen sich durch die gegenseitige Verschwenkbarkeit der einzelnen Beleuchtungs-Teileinheiten gegeneinander verschiedene Einfallswinkel des Lichts bewerkstelligen.

Gemäß einer bevorzugten Ausgestaltung der Erfindung sind die einzelnen Beleuchtungseinheiten zueinander symmetrisch bezüglich der Längsachse der Endoskopoptik angeordnet. Es können also bevorzugt zwei, aber auch mehrere Beleuchtungseinheiten vorhanden sein, die vorzugsweise symmetrisch bezüglich der Längsachse der Endoskopoptik zueinander angeordnet sind und somit im Querschnitt (senkrecht zur Längsachse der Endoskopoptik) Kreissektoren darstellen.

Um eine gleichmäßige Ausleuchtung des Untersuchungsgebiets zu gewährleisten, sind die LED-Elemente der Beleuchtungseinheit bzw. der Beleuchtungs-Teileinheiten gemäß der Erfindung vorzugsweise symmetrisch zueinander bezüglich der Endoskopoptik angeordnet.

Gemäß einer ersten praktischen Ausführungsform der Erfindung mit einem am distalen Ende eines Endoskopschaftes angeordneten Beleuchtungssystem wird vorgeschlagen, dass die Endoskopoptik im Bereich der verschwenkbaren Lagerung der Beleuchtungs-Teileinheiten am Endoskopschaft angeordnet ist, so dass die Endoskopoptik in etwa in einer Ebene mit den Beleuchtungs-Teileinheiten des Beleuchtungssystems gelagert ist.

Mit einer alternativen Ausführungsform der Erfindung wird vorgeschlagen, dass die Endoskopoptik das distale Ende des Endoskopschaftes bildet, während die Beleuchtungseinheit nach proximal versetzt hinter der Endoskopoptik angeordnet ist.

Zur Ableitung der von den LED-Elementen der Beleuchtungseinheit erzeugten Wärme wird mit der Erfindung weiterhin vorgeschlagen, dass im Endoskopschaft ein wärmeableitendes System, insbesondere in Form eines elektrischen Leiters, angeordnet ist.

Schließlich wird mit der Erfindung vorgeschlagen, dass die Endoskopoptik mit einem Bilderfassungssystem gekoppelt ist, das nach dem Abschalten der LED-Elemente der Beleuchtungseinheit Gewebe-Fluoreszenzbilder erfassen kann. Dieses zeitaufgelöste Fluoreszenz-Imaging ist insbesondere bei der Verwendung von Blaulicht-LED-Elementen erhältlich, die Blaulicht bei einer Wellenlänge von ca. 405 nm abstrahlen und das Gewebe zu einer Xeno- oder Autofluoreszenz anregen.

Weitere Merkmale und Vorteile der Erfindung ergeben sich anhand der zugehörigen Zeichnung, in der sechs Ausführungsbeispiele eines erfindungsgemäßen Beleuchtungssystems für endoskopische Untersuchungen nur beispielhaft dargestellt sind. In der Zeichnung zeigt:

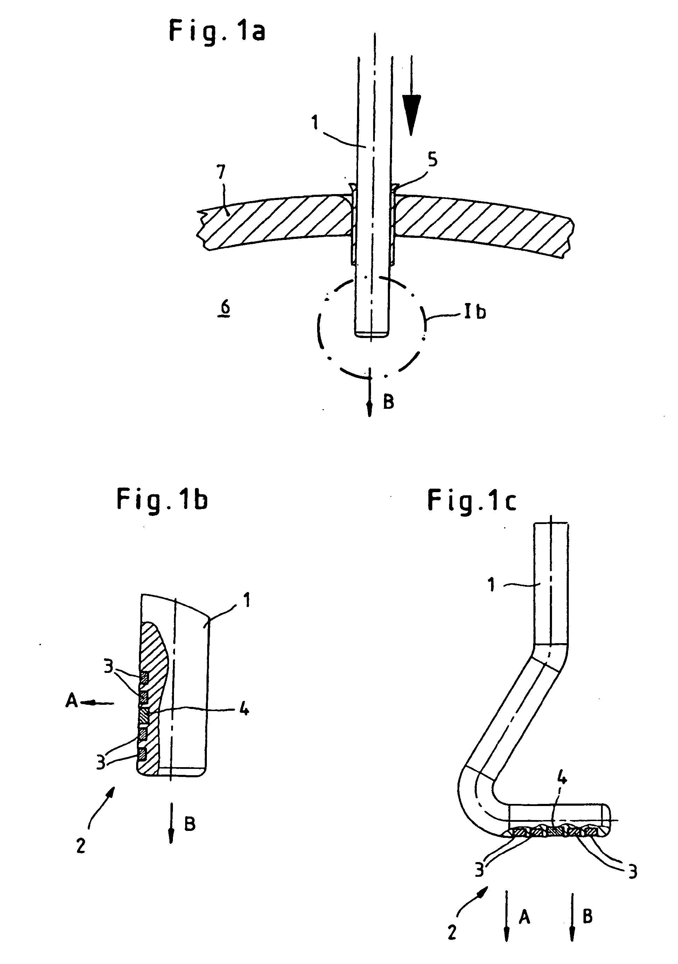

- Fig. 1a: eine Seitenansicht einer ersten Ausführungsform eines erfindungsgemäßen Beleuchtungssystems beim Einführen in das Untersuchungsgebiet;
- Fig.1b: eine vergrößerte und teilweise geschnittene Ansicht des Details Ib gemäß Fig. 1a;
- Fig. 1 c: eine Seitenansicht des Beleuchtungssystems gemäß Fig. 1a im eingeführten Zustand;
- Fig. 2a: eine Seitenansicht einer zweiten Ausführungsform eines erfindungsgemäßen Beleuchtungssystems in der Einführungsposition;
- Fig. 2b: eine teilweise geschnittene Ansicht der Darstellung gemäß Fig. 2a im eingeführten Zustand;
- Fig. 2c: eine Ansicht von unten der Darstellung gemäß Fig. 2b;
- Fig. 3a: eine Seitenansicht einer dritten Ausführungsform eines erfindungsgemäßen Beleuchtungssystems in der Einführungsposition;
- Fig. 3b: eine teilweise geschnittene Ansicht der Darstellung gemäß Fig. 3a im eingeführten Zustand;
- Fig. 3c: eine Ansicht von unten der Darstellung gemäß Fig. 3b;
- Fig. 4a: eine Seitenansicht einer vierten Ausführungsform eines erfindungsgemäßen Beleuchtungssystems in der Einführungsposition;
- Fig. 4b: eine teilweise geschnittene Ansicht der Darstellung gemäß Fig. 4a im eingeführten Zustand;
- Fig. 4c: eine Ansicht von unten der Darstellung gemäß Fig. 4b;
- Fig. 5a: eine Seitenansicht einer fünften Ausführungsform eines erfindungsgemäßen Beleuchtungssystems in der Einführungsposition;
- Fig. 5b: eine um 90° gedrehte Seitenansicht der Darstellung gemäß Fig. 5a;
- Fig. 5c: eine perspektivische Ansicht der Darstellung gemäß Fig. 5a und 5b im eingeführten Zustand;
- Fig. 6a: eine perspektivische Ansicht einer sechsten Ausführungsform eines erfindungsgemäßen Beleuchtungssystems im eingeführten Zustand und
- Fig. 6b: eine teilweise geschnittene Seitenansicht der Darstellung gemäß Fig. 6a.

Die in den Abbildungen Fig. 1a bis Fig. 6b dargestellten Beleuchtungssysteme dienen zur Ausleuchtung endoskopischer Untersuchungsgebiete, unabhängig davon, ob es sich um einen Einsatz in der medizinischen oder in der technischen Endoskopie handelt.

Bei den dargestellten Ausführungsformen bestehen die Beleuchtungssysteme aus einer am distalen Ende eines Endoskopschaftes 1 angeordneten Beleuchtungseinheit 2, die ihrerseits mindestens zwei LED-Elemente 3 aufweist. Zum Betrachten des mittels der Beleuchtungseinheit 2 ausgeleuchteten Untersuchungsgebiets ist am distalen Ende des Endoskopschaftes 1 weiterhin eine Endoskopoptik 4 angeordnet, die klassisch als Relaislinsensystem oder auch als CCD- oder CMOS-Bildsensor zur Video-Bildaufnahme ausgelegt sein kann.

Alle dargestellten Beleuchtungssysteme weisen die Besonderheit auf, dass die Beleuchtungseinheiten 2 nicht an der distalen Stirnfläche des Endoskopschaftes 1, sondern seitlich am distalen Endbereich des Endoskopschaftes 1 angeordnet ist, so dass die Anordnung der LED-Elemente 3 nicht auf die kleine Stirnfläche beschränkt ist.

Wie nachfolgend anhand der verschiedenen Ausführungsformen zur Ausgestaltung der Beleuchtungssysteme näher erläutert wird, sind die LED-Elemente 3 der Beleuchtungseinheiten 2 so an den Endoskopschäften 1 angeordnet, dass die Abstrahlrichtung der Beleuchtungseinheiten 2 zwischen einer Richtung im Wesentlichen senkrecht zur Betrachtungsrichtung der Endoskopoptik 4 beim Einführen in das Untersuchungsgebiet und einer Richtung im Wesentlichen in Betrachtungsrichtung B der Endoskopoptik 4 nach dem Einführen in das Untersuchungsgebiet verstellbar sind.

Die Abbildung Fig. 1a zeigt das Einführen eines Endoskopschaftes 1 mittels einer Trokarhülse 5 in ein Untersuchungsgebiet, beispielsweise den Bauchraum 6 eines Patienten. Zu diesem Zweck wird der Endoskopschaft 1 in der dargestellten langgestreckten geraden Stellung über die in der Bauchdecke 7 angeordnete Trokarhülse 5 in den Bauchraum 6 eingeführt. Diese gerade Einführrichtung entspricht der eigentlichen Betrachtungsrichtung B der Endoskopoptik 4.

Wie aus der in Fig. 1b dargestellten Schnittdarstellung des distalen Endes des Endoskopschaftes 1 gemäß Fig. 1a ersichtlich ist die aus vier LED-Elementen 3 bestehende Beleuchtungseinheit 2 abseits der distalen Stirnfläche am Endoskopschaftes 1 so angeordnet, dass die Abstrahlrichtung A der LED-Elemente 3 der Beleuchtungseinheit 2 beim Einführen in eine Richtung weist, die im Wesentlichen senkrecht zur Betrachtungsrichtung B der Endoskopoptik 4 ausgerichtet ist, die diese bei der Betrachtung des Untersuchungsgebiets einnimmt.

Wie weiterhin aus Fig. 1b ersichtlich, ist die dargestellte Beleuchtungseinheit 2 so aufgebaut, dass die LED-Elemente 3 symmetrisch zueinander bezüglich der als opto-elektronischer Bildsensor ausgebildeten Endoskopoptik 4 angeordnet sind.

Zum Betrachten und Untersuchen des Untersuchungsgebiets ist zumindest der die Beleuchtungseinheit 2 beinhaltende distale Bereich des Endoskopschaftes 1 nach dem Einführen in das Untersuchungsgebiet in die in Fig. 1c dargestellte Stellung verschwenkbar, in der die Abstrahlrichtung A der LED-Elemente 3 der Beleuchtungseinheit 2 im Wesentlichen der Betrachtungsrichtung B der Endoskopoptik 4 entspricht. Aufgrund der aus Fig. 1c ersichtlichen symmetrischen Anordnung der LED-Elemente 3 in Reihe um die Endoskopoptik 4 herum ist es möglich, eine viel größere Leistungsdichte zur Ausleuchtung des Untersuchungsgebiets zu erzielen, als dies mit der aus dem Stand der Technik bekannten Anordnung der LED-Elemente 3 an der kleinen distalen Stirnfläche des Endoskopschaftes 1 möglich ist.

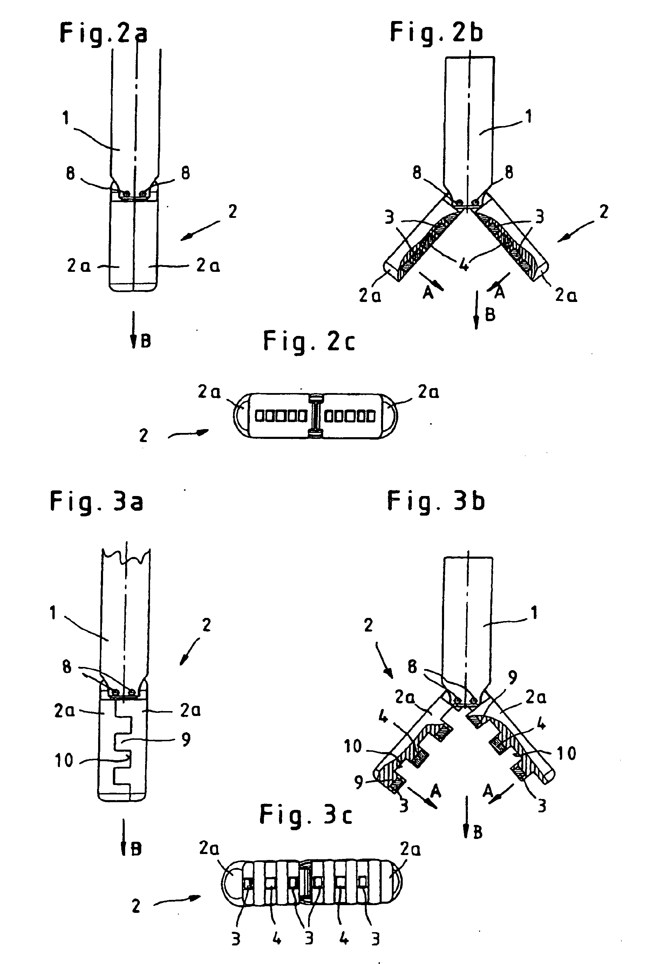

Die in den Abbildungen Fig. 2a bis Fig. 4c dargestellten Ausführungsformen zur Ausbildung von Beleuchtungssystemen für endoskopische Untersuchungen unterscheiden sich von der zuvor beschriebenen Ausgestaltungsform gemäß Fig. 1a bis Fig. 1c dadurch, dass die Beleuchtungseinheiten 2 der dargestellten drei alternativen Konstruktionsformen jeweils aus zwei Beleuchtungs-Teileinheiten 2a bestehen, die um Anlenkpunkte 8 gegeneinander verschwenkbar am Endoskopschaft 1 gelagert sind. Alternativ ist es jedoch auch möglich, drei, vier oder mehr Beleuchtungs-Teileinheiten 2a vorzusehen.

Bei der in Fig. 2a bis Fig. 2c dargestellten zweiten Ausführungsform ist der distale Bereich des Endoskopschaftes 1 zur Ausbildung der zwei Beleuchtungs-Teileinheiten 2a in Axialrichtung des Endoskopschaftes 1 geteilt ausgebildet. Die beiden um jeweils einen Anlenkpunkt 8 verschwenkbaren Beleuchtungs-Teileinheiten 2a weisen bei dieser Ausführungsform jeweils vier LED-Elemente 3 sowie eine als opto-elektronischer Bildsensor ausgebildete Endoskopoptik 4 auf, wobei die LED-Elemente 3 wiederum symmetrisch um die Endoskopoptik 4 angeordnet sind, um eine gleichmäßige Ausleuchtung des Untersuchungsgebiets zu gewährleisten.

Bei dieser Ausführungsform, bei der mehrere distale Endoskopoptiken 4 verwendet werden, die über Lichtleitfasern mit einem Wiedergabegerät (Bildschirm) verbunden sind, werden entweder mehrere einzelne Bilder aus jeweils anderer Blickperspektive angezeigt, wobei gegebenenfalls auch umschaltbar nur einzelne Bilder anzeigbar sind, oder aber es wird ein Stereoblick aus zwei oder mehreren einzelnen Bildern gebildet. Diese letztgenannte Alternative erzielt Bilder mit einer sehr guten dreidimensionalen bzw. Tiefen-Wirkung.

Die Abbildung Fig. 2a zeigt die gestreckte gerade Einführstellung des Endoskopschaftes 1, in der die mit den LED-Elementen 3 sowie den Endoskopoptiken 4 versehenen ebenen Oberflächen der beiden Beleuchtungs-Teileinheiten 2a geschlossen fest aneinander liegen und eine im Wesentlichen bündige distale Verlängerung des Endoskopschaftes 1 bilden.

Zum Überführen in die in Fig. 2b und 2c dargestellten Untersuchungsstellungen werden die Beleuchtungs-Teileinheiten 2a, beispielsweise über ein im Endoskopschaft 1 gelagertes Betätigungselement, insbesondere eine Zug-/Druckstange oder einen Bowdenzug, um die Anlenkpunkte 8 verschwenkt, bis die Abstrahlrichtung A der LED-Elemente 3 der Beleuchtungs-Teileinheiten 2a im Wesentlichen der Betrachtungsrichtung B der Endoskopoptik 4 entspricht. Die Abbildung Fig. 2b zeigt eine zu Untersuchungszwecken verwendbare Übergangsstellung, in der die LED-Elemente 3 aus verschiedenen Einfallswinkeln das Untersuchungsgebiet ausleuchten.

Die in den Abbildungen Fig. 3a bis 3c dargestellte dritte Ausführungsform unterscheidet sich von der zuvor beschriebenen Ausführungsform gemäß den Abbildungen Fig. 2a bis 2c dadurch, dass die mit den LED-Elementen 3 sowie den Endoskopoptiken 4 versehenen Oberflächen der beiden Beleuchtungs-Teileinheiten 2a nicht eben, sondern mit Vorsprüngen 9 und Ausnehmungen 10 versehen kammartig ausgebildet sind. Wie aus Fig. 3a ersichtlich, sind die Vorsprünge 9 und Ausnehmungen 10 der beiden Beleuchtungs-Teileinheiten 2a dabei so angeordnet, dass diese in der Einführstellung des Endoskopschaftes formschlüssig ineinander greifen und die beiden Beleuchtungs-Teileinheiten 2a wiederum eine im Wesentlichen bündige distale Verlängerung des Endoskopschaftes 1 bilden.

Die LED-Elemente 3 sowie die opto-elektronischen Bildsensoren der Endoskopoptiken 4 sind bei dieser Ausführungsform jeweils am freien Ende der Vorsprünge 9 angeordnet, wobei die dargestellte Ausführungsform je Beleuchtungs-Teileinheiten 2a jeweils zwei LED-Elemente 3 sowie eine mittig zwischen den LED-Elementen 3 angeordnete, als opto-elektronischen Bildsensor ausgebildete Endoskopoptik 4 umfasst.

Alternativ zu den in den Abbildungen Fig. 2a bis 3c dargestellten Ausführungsformen, bei denen die opto-elektronischen Bildsensoren der Endoskopoptiken 4 nur auf den Beleuchtungs-Teileinheiten 2a angeordnet sind, ist es selbstverständlich auch möglich, wenigstens einen opto-elektronischen Bildsensor als einzige, oder zusätzliche Endoskopoptik 4 im Bereich der verschwenkbaren Lagerung der Beleuchtungs-Teileinheiten 2a an den Anlenkpunkten 8 anzuordnen.

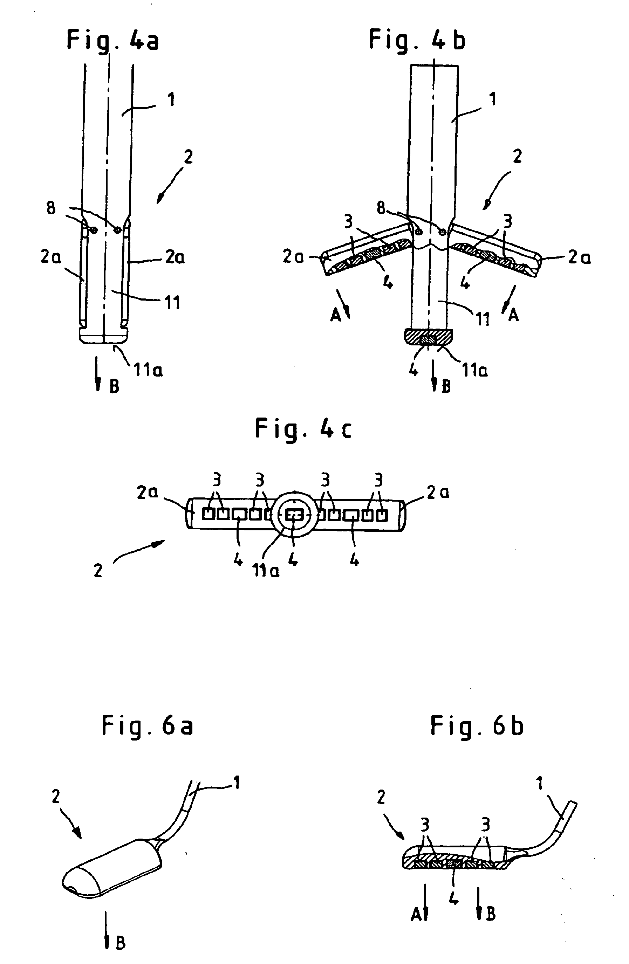

Die Abbildungen Fig. 4a bis 4c zeigen eine weitere Ausgestaltungsform, bei der die Beleuchtungseinheit 2 wiederum aus zwei gegeneinander um Anlenkpunkte 8 verschwenkbaren Beleuchtungs-Teileinheiten 2a besteht. Bei dieser Ausgestaltungsform wird das distale Ende des Endoskopschaftes 1 durch einen brückenförmigen Steg 11 gebildet, der die beiden Beleuchtungs-Teileinheiten 2a in der in Fig. 4a dargestellten Einführstellung überspannt.

Wie insbesondere aus Fig. 4b ersichtlich, sind die beiden Beleuchtungs-Teileinheiten 2a zur Ausleuchtung des Untersuchungsgebiets seitlich aus dem Steg 11 herausklappbar an den Anlenkpunkten 8 gelagert. Die beiden verschwenkbaren Beleuchtungs-Teileinheiten 2a weisen bei dieser Ausführungsform jeweils vier LED-Elemente 3 sowie eine als opto-elektronischen Bildsensor ausgebildete Endoskopoptik 4 auf, wobei die LED-Elemente 3 wiederum symmetrisch um die Endoskopoptik 4 angeordnet sind, um eine gleichmäßige Ausleuchtung des Untersuchungsgebiets zu gewährleisten.

Zusätzlich ist bei dieser Ausgestaltungsform ein weiterer als Endoskopoptik 4 dienender opto-elektronischer Bildsensor auf einer distalen Stirnfläche 11a des Steges 11 angeordnet.

In der in Fig. 4a dargestellten geraden Einführstellung überragt die Stirnfläche 11a des Steges 11 die Beleuchtungs-Teileinheiten 2a in die Ausklapprichtung der Beleuchtungs-Teileinheiten 2a derart, dass die Stirnfläche 11a des Steges 11 den Endoskopschaft 1 im Wesentlichen bündig an die Außenflächen der beiden Beleuchtungs-Teileinheiten 2a anschließend verlängert.

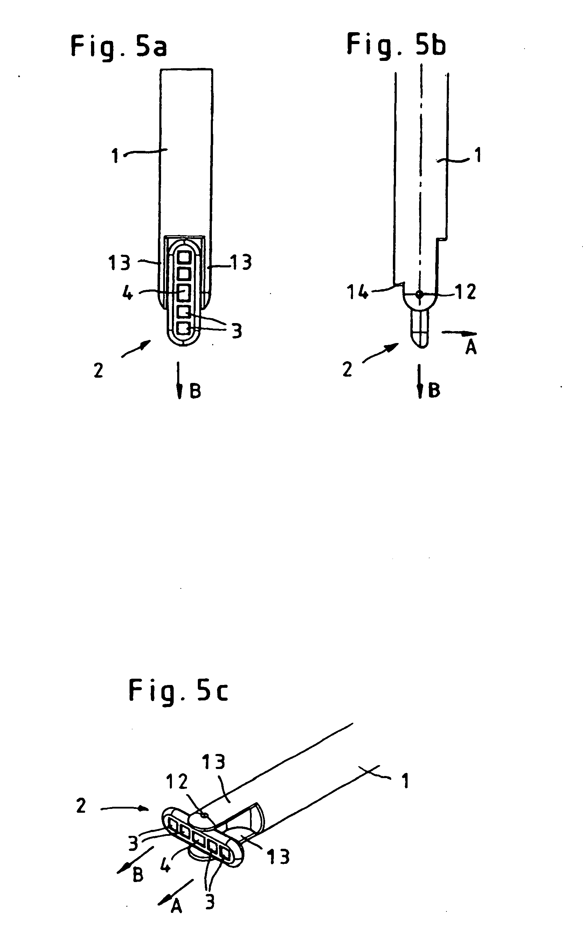

Bei der in Fig. 5a bis 5c dargestellten fünften Ausführungsform ist die einteilig ausgebildete Beleuchtungseinheit 2 um eine Schwenkachse 12 verschwenkbar am distalen Ende des Endoskopschaftes 1 gelagert. Das distale Ende des Endoskopschaftes 1 ist zu diesem Zweck zweiarmig so ausgebildet, dass die Beleuchtungseinheit 2 um die Schenkachse 12 verschwenkbar zwischen den beiden distalen Armen 13 des Endoskopschaftes 1 gelagert ist.

In der in Fig. 5a und 5b dargestellten Einführstellung ist die Beleuchtungseinheit 2 so verschwenkt, dass sie in Axialrichtung des Endoskopschaftes 1 ausgerichtet ist und so mit dem Endoskopschaft 1 in gerader Stellung in das Untersuchungsgebiet eingeführt werden kann.

In der in das Untersuchungsgebiet eingeführten Stellung wird die Beleuchtungseinheit 2, wie aus Fig. 5c ersichtlich, um 90° verschwenkt, bis diese quer zur Axialrichtung des Endoskopschaftes 1 ausgerichtet ist. Ein am Endoskopschaft 1 ausgebildeter Anschlag 14 begrenzt dabei einerseits den Verschwenkwinkel der Beleuchtungseinheit 12 und erlaubt darüber hinaus auch nur das Verschwenken in eine Richtung um die Schwenkachse 12.

Wie weiterhin aus Fig. 5a und 5c ersichtlich, weist die Beleuchtungseinheit 2 bei dieser Ausführungsform vier LED-Elemente 3 sowie eine als opto-elektronischen Bildsensor ausgebildete Endoskopoptik 4 auf, wobei die LED-Elemente 3 wiederum symmetrisch um die Endoskopoptik 4 angeordnet sind, um eine gleichmäßige Ausleuchtung des Untersuchungsgebiets zu gewährleisten.

Alternativ zu der in den in den Abbildungen Fig. 5a bis 5c dargestellten Ausführungsform sind ähnliche Ausgestaltungsformen realisierbar, bei denen proximal von der um 90° verschwenkbaren Beleuchtungseinheit 2 eine oder mehrere weitere Beleuchtungseinheiten 2 um Schwenkachsen 12 verschwenkbar im Endoskopschaft 1 gelagert sind, wobei diese weiteren Beleuchtungseinheiten 2 dann in der Regel jeweils nur mit LED-Elementen 3 zur Ausleuchtung des Untersuchungsgebiets ausgestattet sind und zueinander um jeweils 180° versetzt im Endoskopschaft 1 gelagert sind. Um das Untersuchungsgebiet auch mittels eines Stereoblicks untersuchen zu können, ist es erforderlich wenigstens eine der zusätzlichen Beleuchtungseinheiten 2 zusätzlich mit einer Endoskopoptik 4 auszustatten.

Bei der in Fig. 6a und 6b dargestellten sechsten Ausführungsform eines Beleuchtungssystems bildet die Beleuchtungseinheit 2, ähnlich wie bei der Darstellung gemäß Fig. 1a bis 1c, das distale Ende des Endoskopschaftes 1, der in diesem Fall jedoch als bloßer Leiter für die Versorgung der LED-Elemente 3 sowie der Endoskopoptik 4 ausgebildet ist. Auch bei dieser Ausgestaltungsform weist die Beleuchtungseinheit 2 vier LED-Elemente 3 sowie eine als opto-elektronischen Bildsensor ausgebildete Endoskopoptik 4 auf, wobei die LED-Elemente 3 wiederum symmetrisch um die Endoskopoptik 4 angeordnet sind.

In der nicht dargestellten Einführstellung bildet die Beleuchtungseinheit 2 die gestreckte gerade Verlängerung des Endoskopschaftes 1. Erst im Untersuchungsgebiet wird die Beleuchtungseinheit 2 dann so gegenüber dem Endoskopschaft 1 abgewinkelt, wie dies in Fig. 6a und 6b dargestellt ist.

Wie zuvor dargestellt und beschrieben weisen alle Beleuchtungssysteme weisen die Besonderheit auf, dass die Beleuchtungseinheiten 2 nicht an der distalen Stirnfläche des Endoskopschaftes 1, sondern seitlich am distalen Endbereich des Endoskopschaftes 1 angeordnet ist, so dass die Anordnung der LED-Elemente 3 nicht auf die kleine Stirnfläche beschränkt ist. Zu diesem Zweck sind die LED-Elemente 3 der Beleuchtungseinheiten 2 so an den Endoskopschäften 1 angeordnet, dass die Abstrahlrichtung der Beleuchtungseinheiten 2 zwischen einer Richtung im Wesentlichen senkrecht zur Betrachtungsrichtung der Endoskopoptik 4 beim Einführen in das Untersuchungsgebiet und einer Richtung im Wesentlichen in Betrachtungsrichtung B der Endoskopoptik 4 nach dem Einführen in das Untersuchungsgebiet verstellbar sind.

Für die Ausbildung der LED-Elemente 3 werden vorzugsweise LED-Elemente 3 verwendet, die beispielsweise Blaulicht bei einer Wellenlänge von ca. 405 nm abstrahlen und das Gewebe zu einer Xeno- oder Autofluoreszenz anregen. Die Endoskopoptik 4 ist bei dieser Ausgestaltung des zeitaufgelösten Fluoreszenz-Imaging vorteilhafterweise mit einem Bilderfassungssystem gekoppelt ist, das nach dem Abschalten der LED-Elemente 3 der Beleuchtungseinheit 2 Gewebe-Fluoreszenzbilder erfassen kann.

Der Vorteil der LED-Beleuchtung zur intrakorporalen Fluoreszenzanregung besteht darin, dass die LED's 3 im Gegensatz zu Kurzbogenlampen oder mechanisch geshutterten Konstantlampen sehr genau geschaltet werden können. Somit kann nach einer konstanten Fluoreszenzanregung exakt abgeschaltet und auf Fluoreszenzanregung umgeschaltet werden ("Fluoreszenz-Lifetime-Imaging"). Darüber hinaus kann exakt periodisch angeregt und danach periodisch die Fluoreszierung erzeugt werden.

### Bezugszeichenliste

- 1: Endoskopschaft
- 2: Beleuchtungseinheit
- 2a: Beleuchtungs-Teileinheit
- 3: LED-Element
- 4: Endoskopoptik
- 5: Trokarhülse
- 6: Bauchraum
- 7: Bauchdecke
- 8: Anlenkpunkt
- 9: Vorsprung
- 10: Ausnehmung
- 11: Steg
- 11a: Stirnfläche
- 12: Schwenkachse
- 13: Arm
- 14: Anschlag

- A: Abstrahlrichtung
- B: Betrachtungsrichtung

## Patentansprüche

1. Endoskopschaft (1) mit einem Beleuchtungssystem bestehend aus einer mindestens zwei LED-Elemente (3) aufweisenden Beleuchtungseinheit (2) zur Ausleuchtung eines mittels einer Endoskopoptik (4) zu betrachtenden Untersuchungsgebiets,
**dadurch gekennzeichnet,**
**dass** die LED-Elemente (3) der Beleuchtungseinheit (2) so angeordnet sind dass, die Abstrahlrichtung (A) der Beleuchtungseinheit (2) zwischen einer Richtung im Wesentlichen senkrecht zur Betrachtungsrichtung (B) der Endoskopoptik (4) beim Einführen in das Untersuchungsgebiet und einer Richtung im Wesentlichen in Betrachtungsrichtung (B) der Endoskopoptik (4) nach dem Einführen in das Untersuchungsgebiet verschwenkbar ist.

2. Endoskopschaft (1) nach Anspruch 1, **dadurch gekennzeichnet, dass** die LED-Elemente (3) der Beleuchtungseinheit (2) in Reihe angeordnet sind.

3. Endoskopschaft (1) nach Anspruch 1 oder 2, **dadurch gekennzeichnet, dass** die Beleuchtungseinheit (2) abseits der distalen Stirnfläche am distalen Ende eines Endoskopschaftes (1) angeordnet ist.

4. Endoskopschaft (1) nach einem der Ansprüche 1 bis 3, **dadurch gekennzeichnet, dass** die Beleuchtungseinheit (2) aus mehreren mit LED-Elementen (3) ausgestatteten Beleuchtungs-Teileinheiten (2a) besteht.

5. Endoskopschaft (1) nach Anspruch 4, **dadurch gekennzeichnet, dass** die Beleuchtungs-Teileinheiten (2a) gegeneinander verschwenkbar am Endoskopschaft (1) gelagert sind.

6. Endoskopschaft (1) nach einem der Ansprüche 1 bis 5, **dadurch gekennzeichnet, dass** die LED-Elemente (3) der Beleuchtungseinheit (2) symmetrisch zur Endoskopoptik (4) angeordnet sind.

7. Endoskopschaft (1) nach Anspruch 5, **dadurch gekennzeichnet, dass** die Endoskopoptik (4) im Bereich der verschwenkbaren Lagerung der Beleuchtungs-Teileinheiten (2a) am Endoskopschaft (1) angeordnet ist.

8. Endoskopschaft (1) nach Anspruch 5, **dadurch gekennzeichnet, dass** die Endoskopoptik (4) das distale Ende des Endoskopschaftes (1) bildet.

9. Endoskopschaft (1) nach einem der Ansprüche 3 bis 8, **dadurch gekennzeichnet, dass** im Endoskopschaft (1) ein wärmeableitendes System, insbesondere in Form eines elektrischen Leiters, angeordnet ist.

10. Endoskopschaft (1) nach einem der Ansprüche 1 bis 9, **dadurch gekennzeichnet, dass** die LED-Elemente (3) Licht im Blauen Lichtbereich, vorzugsweise bei ca. 405 nm, abstrahlen.

11. Endoskopschaft (1) nach einem der Ansprüche 1 bis 10, **dadurch gekennzeichnet, dass** die LED-Elemente (3) zur konstanten oder periodischen Fluoreszenzanregung von Gewebe einsetzbar sind.

12. Endoskopschaft (1) nach einem der Ansprüche 1 bis 11, **dadurch gekennzeichnet, dass** die Endoskopoptik (4) mit einem Bilderfassungssystem gekoppelt ist, das nach dem Abschalten der LED-Elemente (3) der Beleuchtungseinheit (2) Gewebe-Fluoreszenzbilder erfassen kann.

## Claims

1. Endoscope shaft (1) with an illumination system comprising an illumination unit (2) having at least two LED elements (3) for illuminating an examination region which is to be viewed by means of endoscope optics (4),
**characterized**
**in that** the LED elements (3) of the illumination unit (2) are arranged such that the beam direction (A) of the illumination unit (2) can be pivoted between a direction substantially perpendicular to the viewing direction (B) of the endoscope optics (4) during the insertion into the examination region and a direction substantially in the viewing direction (B) of the endoscope optics (4) after insertion into the examination region.

2. Endoscope shaft (1) according to Claim 1, **characterized in that** the LED elements (3) of the illumination unit (2) are arranged in series.

3. Endoscope shaft (1) according to Claim 1 or 2, **characterized in that** the illumination unit (2) is arranged to one side of the distal end face on the distal end of an endoscope shaft (1).

4. Endoscope shaft (1) according to one of Claims 1 to 3, **characterized in that** the illumination unit (2) comprises a plurality of illumination components (2a) equipped with LED elements (3).

5. Endoscope shaft (1) according to Claim 4, **characterized in that** the illumination components (2a) are mounted on the endoscope shaft (1) such that they are pivotable relative to each other.

6. Endoscope shaft (1) according to one of Claims 1 to 5, **characterized in that** the LED elements (3) of the illumination unit (2) are arranged symmetrically with respect to the endoscope optics (4).

7. Endoscope shaft (1) according to Claim 5, **characterized in that** the endoscope optics (4) are arranged on the endoscope shaft (1) in the region of where the illumination components (2a) are mounted in a pivotable fashion.

8. Endoscope shaft (1) according to Claim 5, **characterized in that** the endoscope optics form the distal end of the endoscope shaft (1).

9. Endoscope shaft (1) according to one of Claims 3 to 8, **characterized in that** a thermal dissipation system, in particular in the form of an electrical conductor, is arranged in the endoscope shaft (1).

10. Endoscope shaft (1) according to one of Claims 1 to 9, **characterized in that** the LED elements (3) radiate light in the blue light range, preferably at around 405 nm.

11. Endoscope shaft (1) according to one of Claims 1 to 10, **characterized in that** the LED elements (3) can be used for constant or periodic fluorescence stimulation of tissue.

12. Endoscope shaft (1) according to one of Claims 1 to 11, **characterized in that** the endoscope optics (4) are coupled to an image acquisition system which can acquire tissue fluorescence images after the LED elements (3) of the illumination unit (2) have been switched off.

## Revendications

1. Corps d'endoscope (1) comprenant un système d'éclairage constitué d'une unité d'éclairage (2) présentant au moins deux éléments à diodes électroluminescentes LED (3) pour éclairer une région d'examen à observer au moyen d'une optique d'endoscope (4),
**caractérisé en ce que** les éléments à LED (3) de l'unité d'éclairage (2) sont agencés de façon telle que la direction de rayonnement (A) de l'unité d'éclairage (2) puisse pivoter entre une direction sensiblement perpendiculaire à la direction d'observation (B) de l'optique d'endoscope (4) lors de l'introduction dans la région d'examen, et une direction sensiblement dans la direction d'observation (B) de l'optique d'endoscope (4) après l'introduction dans la région d'examen.

2. Corps d'endoscope (1) selon la revendication 1, **caractérisé en ce que** les éléments à LED (3) de l'unité d'éclairage (2) sont agencés en rangées.

3. Corps d'endoscope (1) selon la revendication 1 ou 2, **caractérisé en ce que** l'unité d'éclairage (2) est agencée en retrait de la face frontale distale à l'extrémité distale d'un corps d'endoscope (1).

4. Corps d'endoscope (1) selon l'une des revendications 1 à 3, **caractérisé en ce que** l'unité d'éclairage (2) est constituée de plusieurs unités partielles d'éclairage (2a) équipées d'éléments à LED (3).

5. Corps d'endoscope (1) selon la revendication 4, **caractérisé en ce que** les unités partielles d'éclairage (2a) sont montées sur le corps d'endoscope (1) de manière à pouvoir pivoter l'une par rapport à l'autre.

6. Corps d'endoscope (1) selon l'une des revendications 1 à 5, **caractérisé en ce que** les éléments à LED (3) de l'unité d'éclairage (2) sont agencés de manière symétrique par rapport à l'optique d'endoscope (4).

7. Corps d'endoscope (1) selon la revendication 5, **caractérisé en ce que** l'optique d'endoscope (4) est agencée dans la zone du montage pivotant des unités partielles d'éclairage (2a) sur le corps d'endoscope (1).

8. Corps d'endoscope (1) selon la revendication 5, **caractérisé en ce que** l'optique d'endoscope (4) forme l'extrémité distale du corps d'endoscope (1).

9. Corps d'endoscope (1) selon l'une des revendications 3 à 8, **caractérisé en ce que** dans le corps d'endoscope (1) est agencé un système d'évacuation de chaleur, notamment sous la forme d'un conducteur électrique.

10. Corps d'endoscope (1) selon l'une des revendications 1 à 9, **caractérisé en ce que** les éléments à LED (3) rayonnent de la lumière dans le domaine de la lumière bleue, de préférence aux alentours de 405 nm.

11. Corps d'endoscope (1) selon l'une des revendications 1 à 10, **caractérisé en ce que** les éléments à LED (3) peuvent être utilisés pour l'excitation constante ou périodique en fluorescence des tissus.

12. Corps d'endoscope (1) selon l'une des revendications 1 à 11, **caractérisé en ce que** l'optique d'endoscope (4) est couplée à un système de relevé d'images qui, après l'arrêt des éléments à LED (3) de l'unité d'éclairage (2), peut relever des images de fluorescence des tissus.
